# EUROPEAN PATENT APPLICATION

(11) **EP 2 778 152 A1**
(43) Date of publication of application: **17.09.2014**
(21) Application number: 12847332.9
(22) Date of filing: 08.11.2012
(51) Int. Cl.: C07C 29/58, C07C 33/46, C07B 61/00

(54) **METHOD FOR PRODUCING 4-HYDROXYMETHYL-2,3,5,6-TETRAFLUOROTOLUENE**

(30) Priority: 10.11.2011 JP 2011246239
(71) Applicant: Sumitomo Chemical Company Limited, Tokyo 104-8260 (JP)
(72) Inventor: MIURA, Masaya, Oita-shi Oita 870-0106 (JP); KITAURA, Takenori, Osaka-shi Osaka 554-8558 (JP)
(74) Representative: Vossius & Partner
(86) International application number: PCT/JP2012/079581
(87) International publication number: WO 2013/069810

(57) **Abstract**

A process for producing 4-hydroxymethyl-2,3,5,6-tetrafluorotoluene by subjecting a 4-hydroxymethyl-2,3,5,6-tetrafluorobenzyl halide to contact with hydrogen in the presence of an alcohol solvent having 1 to 3 carbon atoms and palladium on carbon, wherein the temperature of a mixture of the alcohol solvent having 1 to 3 carbon atoms and the palladium on carbon is kept at 15°C or lower until initiation of the contact of the 4-hydroxymethyl-2,3,5,6-tetrafluorobenzyl halide with the hydrogen.

## Description

### Technical Field

The present invention relates to a process for producing 4-hydroxymethyl-2,3,5,6-tetrafluorotoluene.

### Background Art

4-Hydroxymethyl-2,3,5,6-tetrafluorotoluene is a compound useful as a synthesis intermediate of medicines and agrichemicals (Patent document 1).

### Prior Technological Document

### Patient Document

Patent document 1: US-Patent No. 8039680

### Summary of the Invention

### Problem to be Solved by the Invention

There has been a need for a process for producing 4-hydroxymethyl-2,3,5,6-tetrafluorotoluene in good yield.

### Means for Solving the Problem

The present invention includes the following inventions.
[1] A process for producing 4-hydroxymethyl-2,3,5,6-tetrafluorotoluene by subjecting a 4-hydroxymethyl-2,3,5,6-tetrafluorobenzyl halide to contact with hydrogen in the presence of an alcohol solvent having 1 to 3 carbon atoms and palladium on carbon, wherein the temperature of a mixture of the alcohol solvent having 1 to 3 carbon atoms and the palladium on carbon is kept at 15°C or lower until initiation of the contact of the 4-hydroxymethyl-2,3,5,6-tetrafluorobenzyl halide with the hydrogen.
[2] The process according to [1], wherein the contact of the 4-hydroxymethyl-2,3,5,6-tetrafluorobenzyl halide with the hydrogen is conducted at a temperature in the range of from 15°C, exclusive, to 100°C, inclusive.
[3] Theprocess according to [1] or [2], wherein the alcohol solvent is methanol.
[4] The process according to any one of [1] to [3], wherein the 4-hydroxymfethyl-2,3,5,6-tetrafluorobenzyl halide is 4-hydroxymethyl-2,3,5,6-tetrafluorobenzyl chloride.
[5] The process according to any one of [1] to [4], the process comprising steps (1), (2) and (3), wherein the steps (1), (2) and (3) are conducted in order:
   step (1): a step of preparing a mixture of an alcohol solvent having 1 to 3 carbon atoms and palladium on carbon at 15°c or lower,
   step (2): a step of purging the gas phase in the reaction system with hydrogen while keeping the temperature of the mixture prepared in the step (1) at 15°C or lower,
   step (3): a step of dropping a 4-hydroxymethyl-2,3,5,6-tetrafluorobenzyl halide into the reaction system formed in the step (2) to bring the 4-hydroxymethyl-2,3,5,6-tetrafluorobenzyl halide into contact with the hydrogen.
[6] The process according to any one of [1] to [4], the process comprising steps (4) and (5), wherein the steps (4) and (5) are conducted in order:
   step (4): a step of preparing a mixture of an alcohol solvent having 1 to 3 carbon atoms, palladium on carbon and a 4-hydroxymethyl-2,3,5,6-tetrafluorobenzyl halide at 15°C cr lower,
   step (5): a step of purging the gas phase in the reaction system with hydrogen to bring the 4-hydroxymethyl-2,3,5,6-tetrafluorobenzyl halide into contact with the hydrogen while keeping the temperature of the mixture prepared in the step (4) at 15°C or lower.

### Effect of the Invention

According to the present invention, 4-hydroxymethyl-2,3,5,6-tetrafluorotoluene can be produced in good yield.

### Mode for Carrying Out the Invention

Examples of the 4-hydroxymethyl-2,3,5,6-tetrafluorobenzyl halide include 4-hydroxymethyl-2,3,5,6-tetrafluorobenzyl fluoride, 4-hydroxymethyl-2,3,5,6-tetrafluorobenzyl chloride, 4-hydroxymethyl-2,3,5,6-tetrafluorobenzyl bromide and 4-hydroxymethyl-2,3,5,6-tetrafluorobenzyl iodide, 4-hydroxymethyl-2,3,5,6-tetrafluorobenzyl chloride or 4-hydroxymethyl-2,3,5,6-tetrafluorobenzyl bromide is preferred.

Examples of the alcohol solvent having 1 to 3 carbon atoms include methanol, ethanol, n-propanol and isopropyl alcohol, methanol is preferred.

The use amount of the alcohol solvent having 1 to 3 carbon atoms is preferably 0.5 parts by weight to 20 parts by weight per 1 part by weight of the 4-hydroxymethyl-2,3,5,6-tetrafluorobenzyl halide.

As the palladium on carbon, commercially available products can be usually used. As the palladium on carbon, hydrous palladium on carbon may be used.

The use amount of the palladium on carbon is preferably 0.05 mol% to 5 mol% per 1 mol of the 4-hydroxymethyl-2,3,5,6-tetrafluorobenzyl halide, in terms of the amount of a metal contained in the palladium on carbon.

The hydrogen pressure is usually normal pressure to 1 MPa.

The contact of the 4-hydroxymethyl-2,3,5,6-tetrafluorobenzyl halide with the hydrogen may be subjected in the presence of a base, for neutralizing a by-produced hydrogen halide.

Examples of the base include inorganic bases. Examples of the inorganic base include alkali metal hydroxides such as sodium hydroxide and potassium hydroxide; alkaline earth metal hydroxides such as magnesium hydroxide and calcium hydroxide; alkali metal carbonates such as sodium carbonate and potassium carbonate; alkaline earth metal carbonates such as magnesium carbonate and calcium carbonate; alkaline earth metal oxides such as magnesium oxide and calcium oxide.

The use amount of the base is preferably 0.1 mol to 5 mol per 1 mol of the 4-hydroxymethyl-2,3,5,6-tetrafluorobenzyl halide.

The contact of the 4-hydroxymethyl-2,3,5,6-tetrafluorobenzyl halide with hydrogen may be subjected in the presence of other solvent than the alcohol having 1 to 3 carbon atoms.

The other solvent than the alcohol having 1 to 3 carbon atoms is preferably a solvent inactive against the reaction of the 4-hydroxymethyl-2,3,5,6-tetrafluorobenzyl halide with the hydrogen.

Examples of the other solvent than the alcohol having 1 to 3 carbon atoms include aromatic hydrocarbon solvents such as toluene, xylene and chlorobenzene; aliphatic hydrocarbon solvents such as pentane, hexane and heptane; ether solvents such as diethyl ether and methyl tert-butyl ether; ester solvents such as ethyl acetate and butyl acetate, alcohol solvents having 4 or more carbon atoms such as n-butanol and tert-butyl alcohol; water; etc.

The use amount of the other solvent than the alcohol having 1 to 3 carbon atoms is preferably 0 part by weight to 20 parts by weight per 1 part by weight of the 4-hydtroxymethyl-2,3,5,6-tetrafluorobenzyl halide.

The process of the present invention preferably comprises steps (1), (2) and (3), and the steps (1), (2) and (3) are more preferably conducted in order.
step (1): a step of preparing a mixture of an alcohol solvent having 1 to 3 carbon atoms and palladium on carbon at 15°C or lower,
step (2): a step of purging the gas phase in the reaction system with hydrogen while keeping the temperature of the mixture prepared in the step (1) at 15°C or lower,
step (3): a step of dropping a 4-hydroxymethyl-2,3,5,6-tetrafluorobenzyl halide into the reaction system formed in the step (2) to bring the 4-hydroxymethyl-2,3,5,6-tetrafluorobenzyl halide into contact with the hydrogen.

The process of the present invention preferably comprises steps (4) and (5), and the steps (4) and (5) are more preferably conducted in order.
step (4): a step of preparing a mixture of an alcohol solvent having 1 to 3 carbon atoms, palladium on carbon and a 4-hydroxymethyl-2,3,5,6-tetrafluorobenzyl halide at 15°C or lower,
step (5): a step of purging the gas phase in the reaction system with hydrogen to bring the 4-hydroxymethyl-2,3,5,6-tetrafluorobenzyl halide into contact with the hydrogen while keeping the temperature of the mixture prepared in the step (4) at 15°C or lower.

The contact of the 4-hydroxymethyl-2,3,5,6-tetrafluorobenzyl halide with the hydrogen is preferably subjected while continuously introducing hydrogen into the reaction system.

After initiation of the contact of the 4-hydroxymethyl-2,3,5,6-tetrafluorobenzyl halide with the hydrogen, the temperature of the reaction mixture is regulated at a value usually from 15°C, exclusive, to 100°C, inclusive, preferably from 35°C, inclusive, to 100°C, inclusive, more preferably from 35°C, inclusive, to 65°C, inclusive, further preferably from 35°C, inclusive, to 55°C, inclusive, particularly preferably from 35°C, inclusive, to 50°C, exclusive and the reaction is subjected under this condition.

The temperature of the reaction mixture in the terminal period of the contact of the 4-hydroxymethyl-2,3,5,6-tetrafluorobenzyl halide with the hydrogen is preferably from 35°C, inclusive, to 100°C, inclusive, more preferably from 35°C, inclusive, to 65°C, inclusive, further preferably from 35°C, inclusive, to 55°C, inclusive, particularly preferably from 35°C, inclusive, to 50°C, exclusive. After completion of the contact, the reaction mixture may be kept at ordinary temperature if necessary.

Progress of the reaction can be confirmed by usual analysis method such as gas chromatography and liquid chromatography.

The mixture after completion of the reaction contains 4-hydroxymethyl-2,3,5,6-tetrafluorotoluene. 4-Hydroxymethyl-2,3,5,6-tetrafluorotoluene can be isolated, for example, by removing insoluble components such as palladium on carbon from the reaction mixture by filtration, then, adding water and an organic solvent insoluble in water, washing the mixture, and concentrating the resultant organic layer. Examples of the solvent insoluble in water include aromatic hydrocarbon solvents such as toluene, xylene and chlorobenzene; aliphatic hydrocarbon solvents such as pentane, hexane and heptane; ether solvents such as diethyl ether and methyl tert-butyl ether; ester solvents such as ethyl acetate and butyl acetate; etc. The resultant 4-hydroxymethyl-2,3,5,6-tetrafluorotoluene may be further purified by usual method such as distillation, column chromatography and crystallization.

### [EXAMPLES]

The present invention will be illustrated further in detail by examples below. In examples, "%" is by weight unless otherwise stated.

### Example 1

Into a 300 mL flask were charged 20.0 g of 4-hydroxymethyl-2,3,5,6-tetrafluorobenzyl chloride, 50 g of xylene, 50 g of methanol and 2.5 g of magnesium oxide, to give a mixture. A gas phase in the flask was purged with nitrogen, then, the mixture was cooled down to 3°C, 0.5 g of a 50% water-containing product of 5% pal laidum on carbon (containing 2.5% palladium) and 1.5 g of water were added into the flask, and stirred at 5°C for 1 hour, to give a reaction mixture. Thereafter, a gas phase in the flask was purged with hydrogen. The reaction mixture was heated up to 45°C while feeding hydrogen into the flask under normal pressure. While continuously feeding hydrogen into the flask under normal pressure, the reaction mixture was stirred at 45°C for 7 hours, then, the ratio of 4-hydroxymethyl-2,3,5,6-tetrafluorotoluene to 4-hydroxymethyl-2,3,5,6-tetrafluorobenzyl chloride in the reaction mixture was analyzed by a high performance liquid chromatography area percentage method. 4-Hydroxymethyl-2,3,5,6-tetrafluorotoluene was generated in a proportion of 96.7%.

### Example 2

The same reaction as in Example 1 was carried out, excepting that the time of stirring of the reaction mixture at 45°C was changed to 9 hours in Example 1. 4-Hydroxymethyl-2,3,5,6-tetrafluorotoluene was generated in a proportion of 99.9%.

### Example 3

The same reaction as in Example 1 was carried out, excepting that the temperature of the mixture in adding 0.5 g of a 50% water-containing product of 5% pallaidum on carbon (containing 2.5% palladium) and 1.5 g of water into the flask was changed to 10°C, and the time of stirring of the reaction mixture at 45°C was changed to 7.5 hours in Example 1. 4-Hydroxymethyl-2,3,5,6-tetrafluorotoluene was generated in a proportion of 98.9%.

### Comparative Example 1

The same reaction as in Example 1 was carried out, excepting that the temperature of the mixture in adding 0.5 g of a 50% water-containing product of 5% pallaidum on carbon (containing 2.5% palladium) and 1.5 g of water into the flask was changed to 25°C, and the time of stirring of the reaction mixture at 45°C was changed to 8 hours in Example 1. 4-Hydroxymethyl-2,3,5,6-tetrafluorotoluene was generated in a proportion of 84%.

### Example 4

Into a nitrogen-purged 300 mL flask were charged 50 g of methanol, 2.5 g of magnesium oxide and 20 g of xylene, to give a mixture. The resultant mixture was cooled down to -5°C, then, 0.4 g of a 50% water-containing product of 5% pallaidum on carbon (containing 2.5% palladium) and 1.5 g of water were added into the flask. The resultant mixture was stirred at -5°C for 2 hours, then, a gas phase in the flask was purged with hydrogen. While feeding hydrogen into the flask under normal pressure, a mixed solution of 20.0 g of 4-hydroxymethyl-2,3,5,6-tetrafluorobenzyl chloride and 50 g of xylene was dropped into the flask, to give a reaction mixture. Simultaneously with initiation of dropping, the reaction mixture was heated from -5°C to 45°C over a period of 2.5 hours. Dropping was performed over a period of 6 hours. After completion of dropping, the reaction mixture was further stirred at 45°C for 13 hours while continuously feeding hydrogen into the flask under normal pressure, and the ratio of 4-hydroxymethyl-2,3,5,6-tetrafluorotoluene to 4-hydroxymethyl-2,3,5,6-tetrafluorobenzyl chloride in the resulting reaction mixture was analyzed by a high performance liquid chromatography area percentage method. 4-Hydroxymethyl-2,3,5,6-tetrafluorotoluene was generated in a proportion of 99.9%.

### Example 5

Into a 1 L autoclave cooled by immersing in ice water were charged 150 g of methanol, 7.4 g of magnesium oxide, 60 g of xylene, 1.2 g of a 50% water-containing product of 5% pallaidum on carbon (containing 2.5% palladium) and 4.5 g of water, to give a mixture. A gas phase in the autoclave was purged with nitrogen, and the mixture was stirred for 1 hour at an internal pressure of 0.06 MPa. Then, the gas phase in the autoclave was purged with hydrogen, and a mixed solution of 164.6 g of 4-hydroxymethyl-2,3,5,6-tetrafluorobenzyl chloride and 45.5 g of xylene was dropped into the flask while maintaining the internal pressure at 0.07 MPa and feeding hydrogen into the autoclave, to give a reaction mixture. Simultaneously with initiation of dropping, the autoclave was taken cut from ice water, and the reaction mixture was heated up to 45°C over a period of 2.5 hours. Dropping was performed over a period of 7 hours. After completion of dropping, the reaction mixture was further stirred at 45°C for 19 hours while continuously feeding hydrogen into the flask under normal pressure, and the ratio of 4-hydroxymethyl-2,3,5,6-tetrafluorotoluene to 4-hydroxymethyl-2,3,5,6-tetrafluorobenzyl chloride in the resulting reaction mixture was analyzed by a high performance liquid chromatography area percentage method. 4-Hydroxymethyl-2,3,5,6-tetrafluorotoluene was generated in a proportion of 99.6%.

### Comparative Example 2

An atmosphere in a 300 mL flask was purged with nitrogen, and 50 g of methanol, 2.5 g of magnesium oxide, 0.5 g of a 50% water-containing product of 5% pallaidum on carbon (containing 2.5% palladium) and 1.5 g of water were charged therein at 21 °C, to give a mixture. The mixture was stirred at 21°C for 1 hour, then, 20.0 g of 4-hydroxymethyl-2,3,5,6-tetrafluorobenzyl chloride and 50 g of xylene were added to the mixture, to give a reaction mixture. Thereafter, the gas phase in the flask was purged with hydrogen. The internal temperature was raised up to 45°C and the mixture was stirred at 45°C for 9 hours while feeding hydrogen under normal pressure, then, the ratio of 4-hydroxymethyl-2,3,5,6-tetrafluorotoluene to 4-hydroxymethyl-2,3,5,6-tetrafluorobenzyl chloride in the resulting reaction mixture was analyzed by a high performance liquid chromatography area percentage method. 4-Hydroxymethyl-2,3,5,6-tetrafluorotoluene was generated in a proportion of 64%.

### Comparative Example 3

An atmosphere in a 300 mL flask was purged with nitrogen, and 20.0 g of 4-hydroxymethyl-2,3,5,6-tetrafluorobenzyl chloride, 50 g of xylene, 0.5 g of a 50% water-containing product of 5% pallaidum on carbon (containing 2.5% palladium) and 1.5 g of water were charged therein at 25°C, the mixture was stirred at 25°C for 30 minutes, then, 50 g of methanol and 2. 5 g of magnesium oxide were added, and the mixture was further stirred at 25°C for 1 hour. The gas phase in the flask was purged with hydrogen, then, the internal temperature was raised up to 45°C, and the mixture was stirred at 45°C for 8 hours while feeding hydrogen under normal pressure. The ratio of 4-hydroxymethyl-2,3,5,6-tetrafluorotoluene to 4-hydroxymethyl-2,3,5,6-tetrafluorobenzyl chloride in the resulting reaction mixture was analyzed by a high performance liquid chromatography area percentage method. 4-Hydroxymethyl-2,3,5,6-tetrafluorotoluene was generated in a proportion of 84%.

### Industrial Applicability

According to the present invention, 4-hydroxymethyl-2,3,5,6-tetrafluorotoluene can be produced in good yield.

## Claims

1. A process for producing 4-hydroxymethyl-2,3,5,6-tetrafluorotoluene by subjecting a 4-hydroxymethyl-2,3,5,6-tetrafluorobenzyl halide to contact with hydrogen in the presence of an alcohol solvent having 1 to 3 carbon atoms and palladium on carbon, wherein the temperature of a mixture of the alcohol solvent having 1 to 3 carbon atoms and the palladium on carbon is kept at 15°C or lower until initiation of the contact of the 4-hydroxymethyl-2,3,5,6-tetrafluorobenzyl halide with the hydrogen.

2. The process according to Claim 1, wherein the contact of the 4-hydroxymethyl-2,3,5,6-tetrafluorobenzyl halide with the hydrogen is conducted at a temperature in the range of from 15°C, exclusive, to 100°C, inclusive.

3. The process according to Claim 1 or 2, wherein the alcohol solvent is methanol.

4. The process according to any one of Claims 1 to 3, wherein the 4-hydroxymfethyl-2,3,5,6-tetrafluorobenzyl halide is 4-hydroxymethyl-2,3,5,6-tetrafluorobenzyl chloride.

5. The process according to any one of Claims 1 to 4, the process comprising steps (1), (2) and (3), wherein the steps (1), (2) and (3) are conducted in order:
step (1): a step of preparing a mixture of an alcohol solvent having 1 to 3 carbon atoms and palladium on carbon at 15°C or lower,
step (2): a step of purging the gas phase in the reaction system with hydrogen while keeping the temperature of the mixture prepared in the step (1) at 15°C or lower,
step (3): a step of dropping a 4-hydroxymethyl-2,3,5,6-tetrafluorobenzyl halide into the reaction system formed in the step (2) to bring the 4-hydroxymethyl-2,3,5,6-tetrafluorobenzyl halide into contact with the hydrogen.

6. The process according to any one of Claims 1 to 4, the process comprising steps (4) and (5), wherein the steps (4) and (5) are conducted in order:
step (4): a step of preparing a mixture of an alcohol solvent having 1 to 3 carbon atoms, palladium on carbon and a 4-hydroxymethyl-2,3,5,6-tetrafluorobenzyl halide at 15°C or lower,
step (5): a step of purging the gas phase in the reaction system with hydrogen to bring the 4-hydroxymethyl-2,3,5,6-tetrafluorobenzyl halide into contact with the hydrogen while keeping the temperature of the mixture prepared in the step (4) at 15°C or lower.
